# EUROPEAN PATENT SPECIFICATION

(11) **EP 1 119 576 B1**
(45) Date of publication and mention of the grant of the patent: **11.06.2003**
(21) Application number: 99947774.8
(22) Date of filing: 08.10.1999
(51) Int. Cl.: C07H 1/08, C12N 15/10

(54) **ISOLATION METHOD FOR NUCLEIC ACID AND APPARATUS**
VERFAHREN ZUR ISOLIERUNG VON NUKLEINSÄURE UND VORRICHTUNG HIERFÜR
PROCEDE ET SYSTEME D'ISOLEMENT D'UN ACIDE NUCLEIQUE

(30) Priority: 09.10.1998 GB 9822141; 02.08.1999 GB 9918193
(43) Date of publication of application: 01.08.2001
(73) Proprietor: Whatman Bioscience Limited, Abington, Cambridge CB1 6GR (GB)
(72) Inventor: MITCHELL, Andrew, Martin, Milton, Cambridge CB4 6BE (GB); BUTT, Neil, James, Cambridge CB2 2LY (GB); JONES, Christopher, Peter, Heydon, Herts SG8 8PU (GB)
(74) Representative: Hallybone, Huw George
(86) International application number: GB9903357
(87) International publication number: WO00021973

(56) References cited:
- EP-A- 0 814 156
- WO-A-92/07863
- WO-A-98/11989
- US-A- 5 187 083

## Description

### Field of the Invention

The present invention relates to a method for isolating nucleic acid (DNA) from a sample containing nucleated cells.

### Background of the Invention

Whilst relatively rapid and convenient procedures for the purification of nucleic acid (such as DNA) from agarose have been developed, it remains a relatively difficult operation to extract nucleic acid directly from more complex.starting samples such as cells and cell lysates. On the whole, the procedures currently practised to purify nucleic acid from nucleic acid-containing samples comprising cells or cell lysates remain to be time-consuming and labour intensive.

One method proposed to minimise the laborious and time-consuming steps of the known method for isolating nucleic acid from these more complex example is described in EP 0389063. The method disclosed in EP 0389063 involves mixing the cell sample (such as whole blood) with a chaotropic substance and a particulate nucleic acid binding solid phase comprising silica or a derivative thereof. It is well known that, in the presence of a chaotropic substance, nucleic acid is released from cells and binds to silica-based nucleic acid binding solid phases. Subsequently, the mixture is centrifuged to pellet the solid phase with the nucleic acid bound thereto and the supernatant is discarded. The pelleted material is subjected to several washing stages with chaotropic agent and organic solvents. Finally, the DNA is eluted from the solid phase in a low salt buffer.

The method described in EP 0389063 is disadvantageous in that it is a manually intensive, multi-step procedure. In view of the fact that the method involves a number of centrifugation and vessel transfer steps, this method is unsuitable for automation.

US patent No. 5 652 141 (see also WO 92/07863) discloses a method for isolating nucleic acids by lysing intact blood cells in a porous matrix having ion exchange properties.

US Patent Nos. 5,187,083 and 5,234,824 each describe a method for rapidly obtaining substantially pure DNA from a biological sample containing cells. The methods involve gently lysing the membranes of the cells to yield a lysate containing genomic DNA in a high molecular weight form. The lysate is moved through a porous filter to trap selectively the high molecular weight DNA on the filter. The DNA is released from the filter using an aqueous solution.

EP-A-0 814 156 discloses a method for collecting DNA from microbial non-nucleated cells. The cells are lysed on filters in a column and DNA is collected by the chaotropic ion method.

The present invention aims to provide an improved method for isolating nucleic acid from a nucleic acid-containing sample which avoids the use of centrifugation steps and which avoids the requirement of upstream processing of the sample in order to render the nucleic acid amenable to binding to the solid phase.

### Summary of the Invention

According to the present invention, there is provided a method for isolating nucleic acid (DNA) which comprises:
(a) applying a sample comprising nucleated cells containing nucleic acid to a filter, whereby the cells are retained as a retentate and contaminants are removed;
(b) lysing the retentate from step (a) whilst the retentate is retained by the filter to form a cell lysate containing the nucleic acid;
(c) filtering the cell lysate with the filter to retain the nucleic acid and remove remaining cell lysate;
(d) optionally washing the nucleic acid retained by the filter; and
(e) eluting the nucleic acid, wherein the filter composition and dimensions are selected so that the filter is capable of retaining the cells and the nucleic acid.

Preferably, step (b) comprises lysing the retentate whilst it is entrapped within the filter.

The filter composition and dimensions are selected so that the nucleic acid is retained by the filter in step (c) substantially in the absence of ionic interaction, preferably by a physical retarding of the movement of the nucleic acid down the filter. Preferably, the filter composition and dimensions are selected so that the nucleic acid is retained by the filter in step (c) in the form of a web.

Preferably, the nucleic acid is heated to an elevated temperature whilst retained by the filter prior to eluting in step (e).

### Brief Description of the Drawings

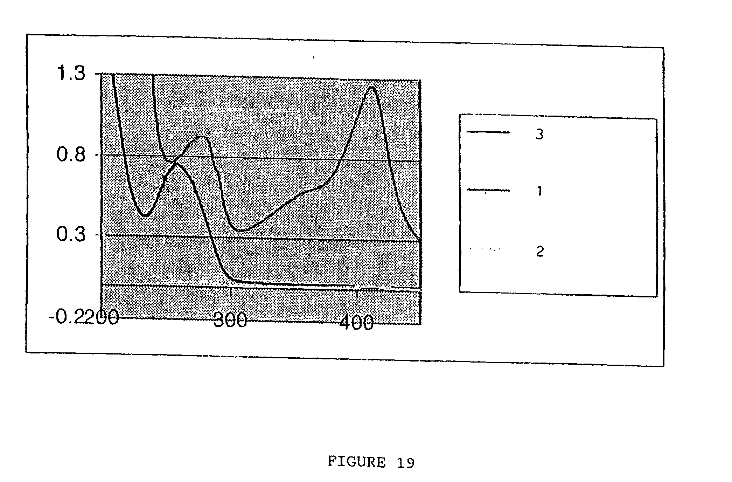

The present invention will now be described in further detail with reference to the accompanying Examples and Experiments and to the attached Figures in which:
- Figure 1: shows an image obtained by scanning electron microscopy (SEM) of the structure of a filter material suitable for use in the present method.
- Figure 2: shows an image obtained by SEM of the retained retentate and the filter.
- Figure 3: shows an image obtained by SEM of the retained retentate and a top portion of the filter.
- Figure 4: shows an image obtained by SEM of the retained retentate and a lower portion of the filter.
- Figure 5: shows an image obtained by SEM of the retentate comprising a damaged white cell which is retained by the filter.
- Figure 6: shows an image obtained by SEM of the nucleic acid which is retained by the filter in the form of web.
- Figure 7: shows a close-up image obtained by SEM of the nucleic acid web as it is retained by the filter.
- Figure 8: shows an image obtained by SEM of the nucleic acid web and the filter after the optional step of heating and/or incubation prior to elution.
- Figure 9: shows an image obtained by SEM of the filter after elution of the nucleic acid.
- Figure 10: shows agarose gel analysis of DNA capture from a stack of filters;
- Figure 11: shows agarose gel analysis of drop-out of DNA levels from various filter arrangements;
- Figure 12: shows agarose gel photograph showing the proportion of DNA eluted from layers of filters within a system;
- Figure 13: shows agarose gel analysis of DNA recovery from filters in the presence of 1M TRIS pH 8.5 and 50% ethanol;
- Figure 14: shows agarose gel analysis of DNA extracted from whole blood and eluted in a range of salt concentrations;
- Figure 15: shows agarose gel analysis of DNA eluted from filters at different elution temperatures;
- Figure 16: shows agarose gel photograph showing the results of altering the time of elution post heating;
- Figure 17: shows agarose gel photograph showing the results of altering the pH of the elution buffer over the range pH 5.5 to 11.0;
- Figure 18: shows a clinical extraction device suitable for use in the present invention;
(a) = Syringe Barrel
(b) = Extraction Cartridge
(c) = Needle
(d) = Section through Extraction Cartridge
(e) = Female Lure
(f) = Filter Material
(g) = Filter retainer
(h) = Male Lure
(i) = Filter support
(j) = EDTA Coated
- Figure 19: shows spectroscopic analysis of solutions eluted from the present column
(1) = waste eluant after step 3
(2) = waste eluant after step 4
(3) = eluted DNA solution
- Figure 20: agarose gel photograph showing relationship between incubation time, temperature and DNA yield and size; and
- Figure 21: agarose gel photograph showing DNA extracted from 500µl clinical whole blood samples using the present method.

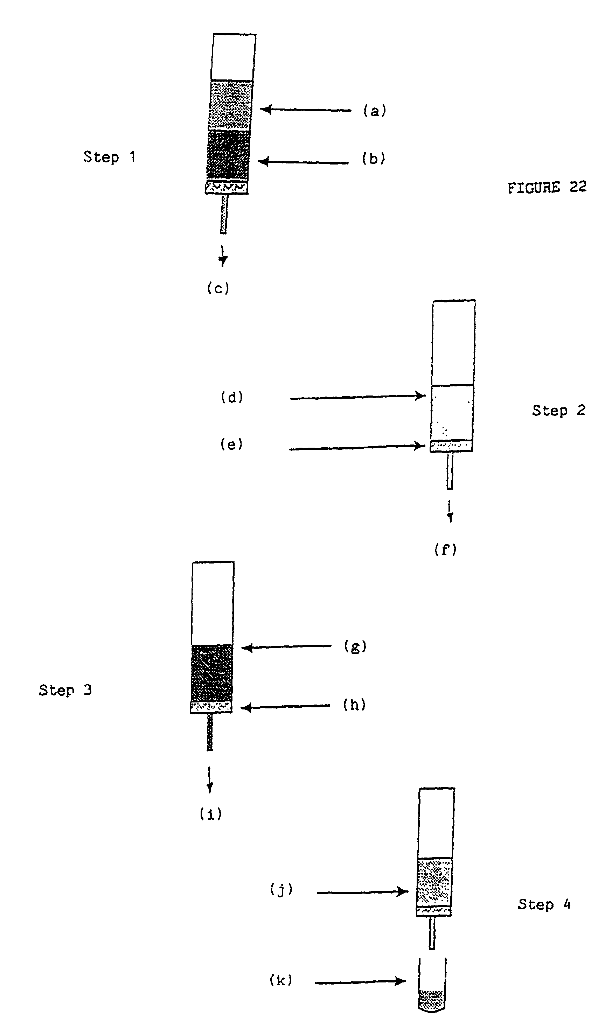

- Figure 22: step-by step diagram of one embodiment of the present method
Step 1: Non-nucleated cell lysis
Step 2: Nucleated cell lysis
Step 3: Wash Step
Step 4: Elution

(a) = 1 volume red cell lysis solution
(b) = 1 volume whole blood
(c) = Supernatant to waste
(d) = 1 volume lysis solution
(e) = gDNA captured within filter
(f) = Supernatant to waste
(g) = 1 volume wash solution
(h) = gDNA captured within filter
(i) = Supernatant to waste
(j) = heat at 90°C for 8 minutes
(k) = add volume H₂O and elute

Traditionally, filters are selected so as to have a pore size and composition which will act as an absolute barrier so as to prevent the material to be filtered from passing through or into the filter material. For example, by selecting a filter material with a particular pore size it is possible to prevent materials with a particle size greater than the pore size from passing through or into the filter material. However, it has been found by the applicant that an improved method for purifying nucleic acid is obtained when a filter material is selected which does not provide an absolute barrier to the cells, but enables the cells to be retained by the filter as a retentate, in particular to pass into the filter material and to become entrapped therein. These steps occur prior to lysing the retentate whilst the retentate is retained by the filter to form a cell lysate containing the nucleic acid. Subsequent to *in situ* lysing of the retentate, the filter is also capable of retaining the nucleic acid but not other cell components.

As a consequence, where the sample comprises whole blood which has been treated with a red blood cell lysis solution, the solution containing red cell debris passes through the filter and may be discarded whilst the white cells containing the nucleic acid are retained by the filter as a retentate. Red cell lysis is not absolutely necessary as the filter will allow intact red cells to pass through. However, inclusion of the red blood cell lysis solution leads to a cleaner final product.

It has been found that the present method substantially improves the yield and purity of the nucleic acid product. Furthermore, the present method provides a quick, simplified, cost effective method for nucleic acid purification that is not manually intensive or technique dependent and does not utilize hazardous chemicals. The nucleic acid produced in accordance with the present invention is capable of multiple downstream processing. Optionally, the nucleic acid retained by the filter may be washed with any suitable wash solution. Preferably, the nucleic acid retained by the filter is washed with a buffer having a pH in the range 5.8 to 10, more preferably in the range 7 to 8. In particular, washing with water or a low salt buffer such as TE⁻¹ (10 mM Tris HCl (pH8) with 100µM EDTA) is preferred. The washing step may occur prior to or at the same time as elution in step (e). Washing increases the yield and purity of the nucleic acid product and ensures that the filter stays damp during incubation. If the filter is allowed to dry the nucleic acid still is recoverable but may be sheared and the yield will be reduced. Where the method is carried out in a column, drying of the filter also may be avoided by using a water vapour retarding or blocking seal such as a rubber bung in the column to reduce evaporation of solution from the filter. The washing step removes any remains of the nuclear material-lysis solution which may be problematic in downstream processing.

It is preferred that the retentate be. lysed whilst entrapped within the filter. However, it should be understood that the method according to the present invention encompasses also an embodiment where substantially all or some of retentate is lysed whilst retained by but not entrapped within the filter.

In one aspect of the present invention, the retentate comprises condensed nuclear material and cell debris. In this aspect, on application of a sample comprising cells containing nucleic acid to the filter, the cell membrane is ruptured or gently "peeled away" to form condensed nuclear material and cell debris which is retained by the filter. It is thought that the condensed nuclear material may comprise intact nuclei.

In another aspect of the present invention, the retentate comprises intact whole cells as well as or instead of condensed nuclear material and cell debris. Advantageously, the intact whole cells may be treated in step (b) whilst retained by the filter by the application of a detergent to the filter. This has the effect of rupturing or "peeling away" the cell membrane to leave condensed nuclear material. The condensed nuclear material is retained by the filter. Preferably the detergent is one of SDS (particularly 0.5% SDS), TWEEN 20 (particularly 1% TWEEN 20), LDS (particularly 1% LDS) or TRITON (particularly 1% TRITON).

Whilst the addition of detergent to the retentate is preferable, the present method may be carried out without the addition of a detergent. However, applying a detergent to the retentate whilst the retentate is retained by the filter increases the yield and purity of the DNA product.

In addition to rupturing the intact whole cells to form condensed nuclear material, the detergent also has the function of washing out protein and haem which may have been retained by the filter.

The retentate does not comprise freed nucleic acid prior to step (b) .

Preferably, the retentate is lysed to form a cell lysate containing nucleic acid in step (b) by the addition low salt buffer. Preferably, the low salt buffer is TE⁻¹ or water. Other suitable lysis solutions include any detergent-containing solutions in which the detergent may be cationic, anionic or neutral. Chaotrope-containing solutions, preferably buffers may also be used. The lysis solution lyses or bursts open the condensed nuclear material to release the nucleic acid. It will be understood by the skilled person, however, that lysing the retentate to form a cell lysate containing nucleic acid also can be achieved by other methods, for example by heating.

The retention or entrapment of the cells and nucleic acid by the filter may arise by virtue of a physical or size-related barrier relating to the dimensions of the filter material including the pore size and depth of the filter, or by other means. Without wishing to be bound by theory, it is thought that the nucleic acid may be associated with the filter rather than bound tightly thereto. It is postulated that nucleic acid-nucleic acid interactions themselves are important in maintaining a sufficiently high cross-sectional area to retard movement of the nucleic acid through the filter.

Preferably, the filter composition and dimensions are selected so that the nucleic acid is retained by the filter in step (c) in the form of a web. For the purpose of the present invention, the term "web" can be taken to include partly or substantially disordered structures, lattice-type structures, mesh-type structures, complex network-type structures, tangle-type structures or knot-type structures. The web may have a loose or open stringy-type structure and may comprise a plurality of strands. The web structure does not involve substantial direct or intimate binding, for example by ionic interactions, of the nucleic acid directly onto the filter.

Advantageously, the filter comprises a plurality of fibres and has a substantially disordered structure. Preferably, the fibre diameters are selected so that the nucleic acid is retained by the filter in step (c) in the form of a web. In accordance with the definition of the term "web" as described above, the structure of the web is such that there is substantially no direct binding, for example by ionic interactions, of the nucleic acid directly onto the fibres. More preferably the fibre diameters are selected so that they are in the range of from 1µm to 15µm, even more preferably in the range of from 1µm to 10µm, most preferably about 10µm.

Filter materials that are suitable for use in the present invention include any material which enables the cells to be retained by the filter as a retentate and the nucleic acid to be retained by the filter, preferably in the form of a web. Suitable materials include glass fibre or any silica-based or derived filters and plastics based filters, for example polyester and polypropylene based filters.

Referring to the filter, its composition and dimensions are selected so that it is capable of retaining the cells and the nucleic acid substantially in the absence of a chaotrope. It has been surprisingly found by the applicant that with certain filter materials, it is possible to isolate nucleic acid in the absence of a chaotrope. This goes against the conventional wisdom of those skilled in the art of the invention.

The filter material is of certain depth that is sufficiently large to entrap the cells and the nucleic acid within the filter, preferably without substantial loss. Accordingly, a filter of a suitable depth may comprise a plurality of filters arranged in series. The number of filters influences the total nucleic acid yield and concentration. Preferably, the plurality of filters is stacked one above the other and is supported by a frit. The present method is scalable so that any surface area of the filter and thus any filter diameter may be used.

One suitable filter for use in the present method is a stack of four Whatman GF/D variant filters. The filters may be stacked into a column of 6mm in diameter and supported on a frit. Various parameters of the GF/D variant filter are set out in Table 1 below.

**Table 1**

| Parameter | Units | Typical Values |
|---|---|---|
| Grammage | g/m² | 115 |
| Thickness @53kPa | µm | 677 |
| Porosity (5oz cylinder) | s/300mIs/in² | 4.7 |
| Tensile (MD) | N/15mm | 5.8 |
| Water Absorption | mg/cm² | 137 |
| Pore Sizes | µm | 4.5 |
| Minimum | | 14.5 |
| Maximum | | 7.9 |
| Mean | | |

It is preferred also that the filter composition and dimensions are selected so that the nucleic acid in step (e) is capable of being eluted at a pH of from pH 5 to 11 or from 5.8 to 10. This is advantageous in the present method because elution of the product nucleic acid is a highly alkaline medium potentially can degrade the product. Accordingly, one preferred pH for elution is from 7 to 9.

The applicants have found that as a consequence of selecting the filter composition and dimensions so as to meet the above requirements, the filter often substantially is not capable of retaining purified DNA when purified DNA is applied to the filter. In addition, the filter is substantially incapable of retaining cells which are lysed off-line and then applied to the filter (an 80% drop in yield is observed as compared with the present method).

Eluting the nucleic acid, in other words releasing the nucleic acid from the filter, may be affected in several ways. The efficiency of elution may be improved by putting energy into the system during an incubation step to release the nucleic acid prior to elution. This may be in the form of physical energy (for example by agitating) or heat energy. The incubation or release time may be shortened by increasing the quantity of energy put into the system. Preferably, heat energy is put into the system by heating the nucleic acid to an elevated temperature for a predetermined time, whilst it is retained by the filter, prior to eluting in step (e). However, elution still may be effected when the nucleic acid has not been heated to an elevated temperature or even has been held at a lowered temperature (as low as 4°C) prior to elution in step (e). More preferably, the nucleic acid is heated to an elevated temperature in the range of 40°C to 125°C, even more preferably in the range of from 80°C to 95°C. Most preferably, the nucleic acid- is heated to an elevated temperature of about 90°C, advantageously for about 10 minutes for a filter having a 6mm diameter. Increasing the filter diameter increases the yield of DNA at any given heating temperature.

It should be noted that predominantly single stranded material will be produced from the present system. However, the ratio of double to single stranded DNA is dependent upon, and can be controlled by, the experimental conditions. Modifying the incubation regime using the parameters of time and temperature will alter this ratio, where a lower elution temperature over a longer time period will produce a high proportion of double stranded DNA. A higher elution temperature over a shorter period of time also will produce a higher proportion of double stranded DNA.

Once the nucleic acid has been heated to an elevated temperature whilst retained by the filter, it is not necessary to maintain the nucleic acid at the elevated temperature during elution. Elution itself may be at any temperature. For ease of processing, it is envisaged that in a preferred embodiment where the nucleic acid is heated to an elevated temperature whilst retained by the filter, elution will be at a temperature lower than the elevated temperature. This is because when heating has been stopped, the temperature of the nucleic acid will fall over time and also will fall as a result of the application of any ambient temperature eluting solution to the filter. Any solution at any pH would be suitable for eluting the nucleic acid from the present filter. Preferred elution solutions include NaOH, Na acetate 1mM to 1M, 10mM MES (pH 5.6), 10mM CAPS (PH 10.4) TE (10mM Tris HCL (pH8) + 1mM EDTA), TE⁻¹, SDS (particularly 0.5% SDS), TWEEN 20 (particularly 1% TWEEN 20), LDS (particularly 1% LDS) or TRITON (particularly 1% TRITON), water and 10mM Tris. All yield approximately the same quantity of nucleic acid. Total yields of nucleic acid are higher when eluted in a high volume of elution solution.

In steps (a) to (d) of the present method, the temperature is usually ambient temperature, typically in the range 5°C to 40°C.

In general, the present method may be applied advantageously to any whole cell suspension. Nucleated cells particularly amenable to the present method include yeast cells and mammalian cells, such as white blood cells, epithelial cells, buccal cells, tissue culture cells and colorectal cells. DNA has been obtained successfully from CEP swabs, saline and sucrose mouthwashes and buffy coat samples.

Where the cells comprise white blood cells, it is preferred that the method further comprises applying whole blood to the solid phase, optionally lysing the red blood cells therefrom, optionally washing the solid phase to remove contaminants and obtaining the cell lysate from the blood cells. The whole blood can be fresh or frozen. Na/EDTA K/EDTA and citrated blood all give similar yields. A 100µl sample of whole blood gives a yield of approximately 2-5µg, a 500µl sample gives a yield of approximately 15-40µg and a 10ml sample gives a yield of approximately 200-400µg.

It is preferred that the nucleic acid comprises a polynucleotide.

Whilst the method is applicable to any nucleic acid, the nucleated cell comprises DNA, especially genomic DNA.

It is preferred that the method be conducted without any centrifugation steps.

It is preferred that the method be conducted substantially in the absence of a chaotrope.

It is believed by the applicants that this method is particularly useful for the extraction of genomic DNA from whole blood. This method can be conducted in a single vessel, and does not require any centrifugation steps, therefore making the method suitable for automation. One suitable method for extracting genomic DNA from a whole blood sample involves the following steps:
i) Whole blood is charged into a column containing one or more (preferably 4 standard depth) of GF/D variant filters (Whatman International Ltd, Maidstone, UK). This arrangement of glass fibre filters has been found to be of a sufficient depth to effect separation of the white blood cells from other components of whole blood cells in the downstream processing, separation of the genomic DNA from other material;
ii) A red blood cell-lysis solution is delivered to the column in order to lyse red blood cells;
iii) The red blood cell-lysis solution is drawn through the filters leaving white blood cells entrapped within the filter;
iv) White cell-lysis solution is delivered to the column;
v) The white cell-lysis solution is drawn through the filters. It is believed that DNA from the white blood cells forms an association with the glass fibre filters. It is apparent that ionic interaction is minimal, and accordingly it appears that there is a physical retarding of the movement of the DNA down the filter;
vi) A low salt buffer is delivered to the column and washed through. The DNA remains associated with the glass fibre filter;
vii) Further low salt buffer is delivered into the column. This is then heated at a temperature and for a sufficient time to release the DNA from the filter. Preferably, the column is heated to a temperature within the range 78-90°C (usually 82°C) for a time of approximately 50 minutes;
viii) DNA is eluted in the low salt buffer. The DNA is of multiplex PCR quality.

Whilst it is indicated in this preferred method that genomic DNA is the desired target compound, it is possible to use the method of the present invention to isolate RNA from an RNA-containing sample.

It will also be appreciated to those skilled in the art of the invention that whole blood may be subjected to a red blood cell-lysis solution in a separate vessel prior to transfer of the mixture to the filter. Typical red blood cell lysis solutions that may be used in the method of the invention include those set out in Table 2.

The method may be practised by using a kit comprising:
(a) an apparatus comprising a filter supported by a support, wherein the filter composition and dimensions are selected so that the filter is capable of retaining the cells and the nucleic acid as described above;
(b) a red cell lysis solution, a solution for rupturing intact whole cells to leave condensed nuclear material, a lysis solution for lysing nuclear material and an elution solution.

The filter may be supported in or on the support or may form an integral part of the support.

The support may be, for example, any tube or column made from plastics, glass or any other suitable material. The filter supported on the support may be held in place by a frit. This would prevent movement of the filter which may occur when the sample comprising cells or any other solution is applied to the filter.

The solutions which may be used in the kit are typically those suitable for use in the method according to the present invention, as described herein.

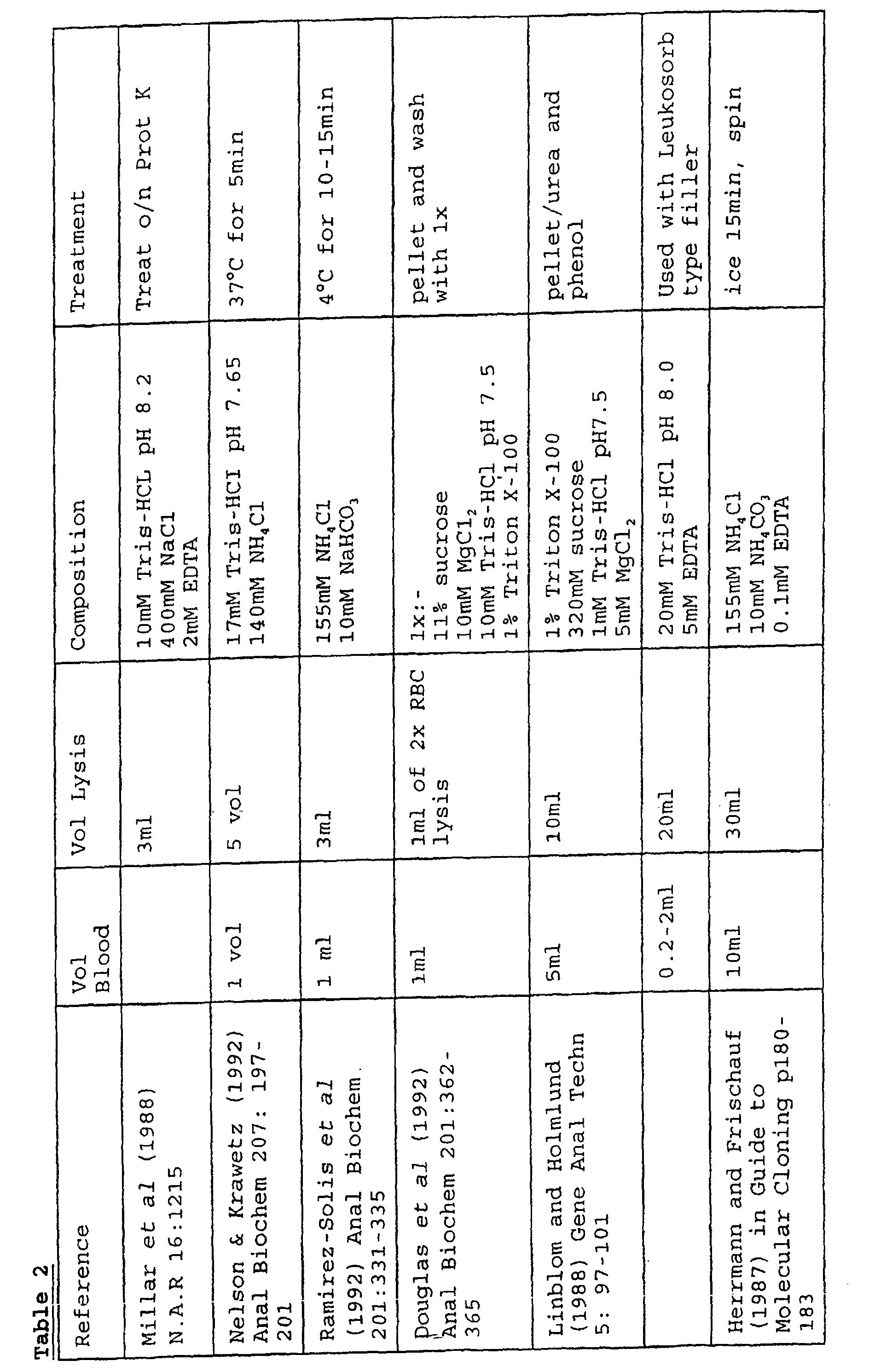

### Examples

### Example 1: Preparation of purified product

DNA extraction from 200µl of human whole blood was carried out using the present method. The protocol was as follows:
1) Add 200µl of whole blood to the column, add 1000µl of red blood cell lysis solution (RBCL), filter to waste.
2) Add 1000µl RBCL filter to waste.
3) Add 1000µl 0.5% SDS, filter to waste.
4) Add 1000µl TE¹, filter to waste.
5) Add 100µl TE¹, filter to waste.
6) Incubate at 90°C for ten minutes.
7) Add 100µl TE¹, filter to capture DNA solution.

The mean DNA yield for the present method is 30-40µg per ml of blood. About 80% of the DNA product is greater than 40kb. The approximate time per cycle is 20 minutes, i.e. significantly faster than presently known methods.

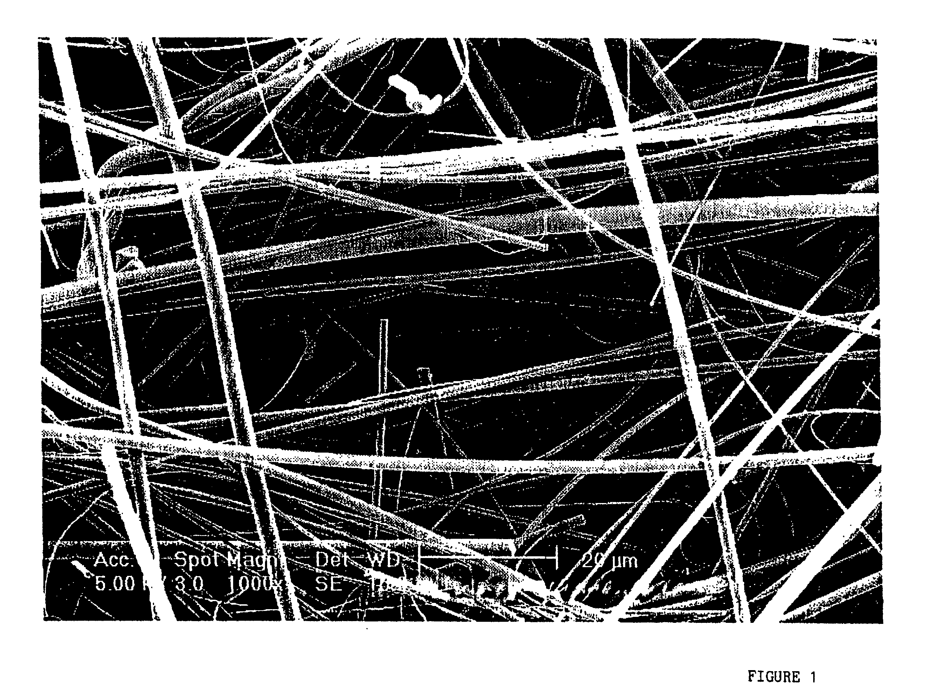

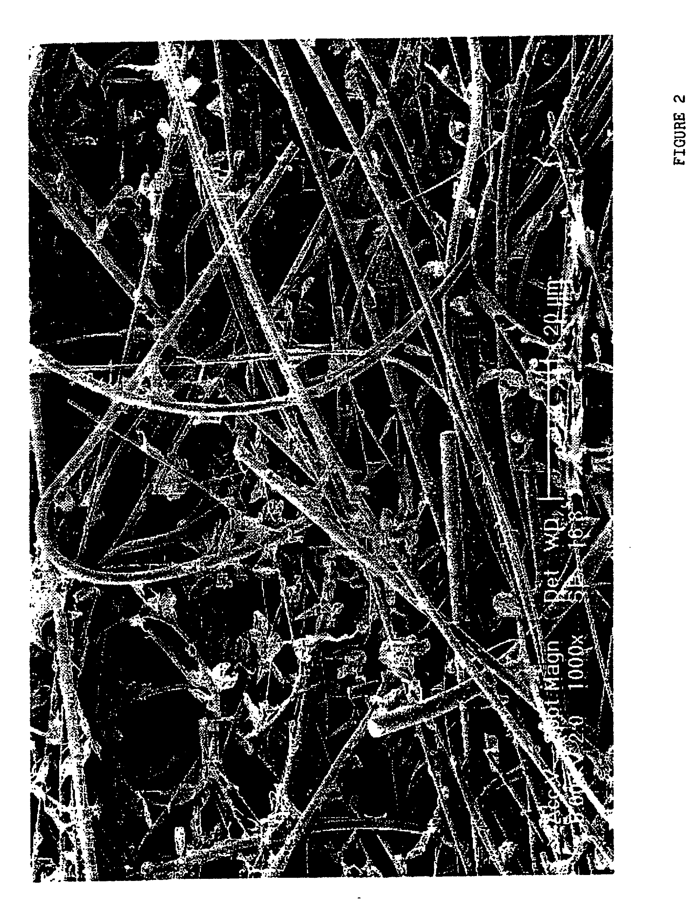

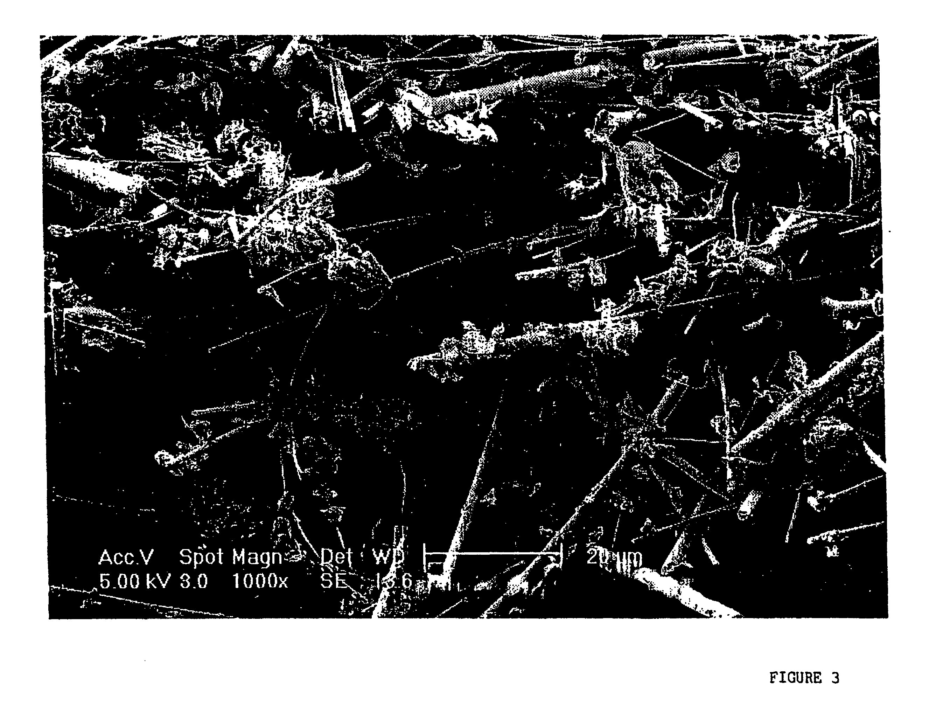

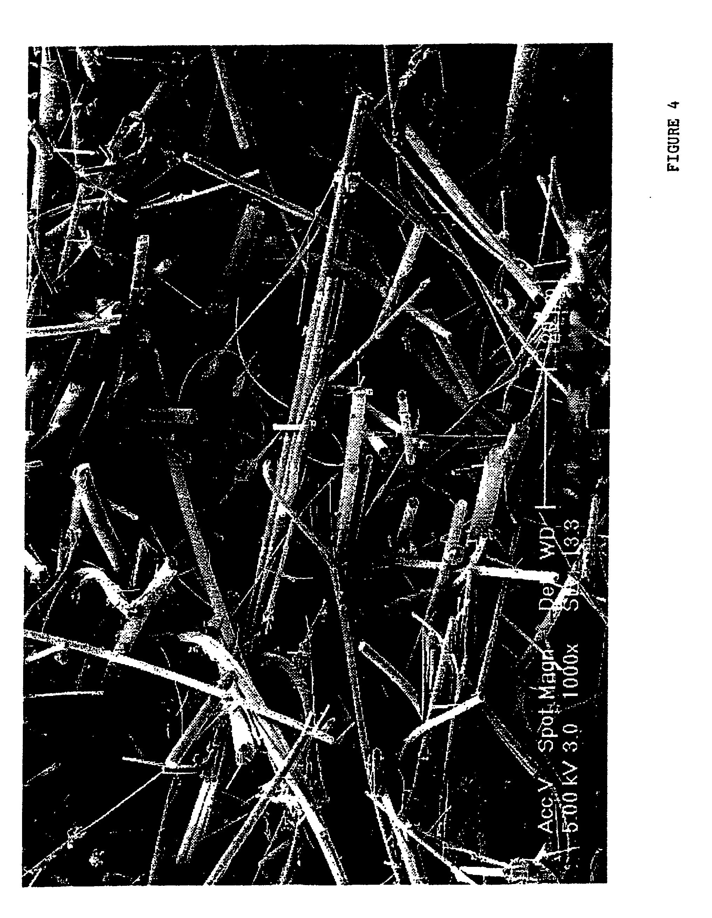

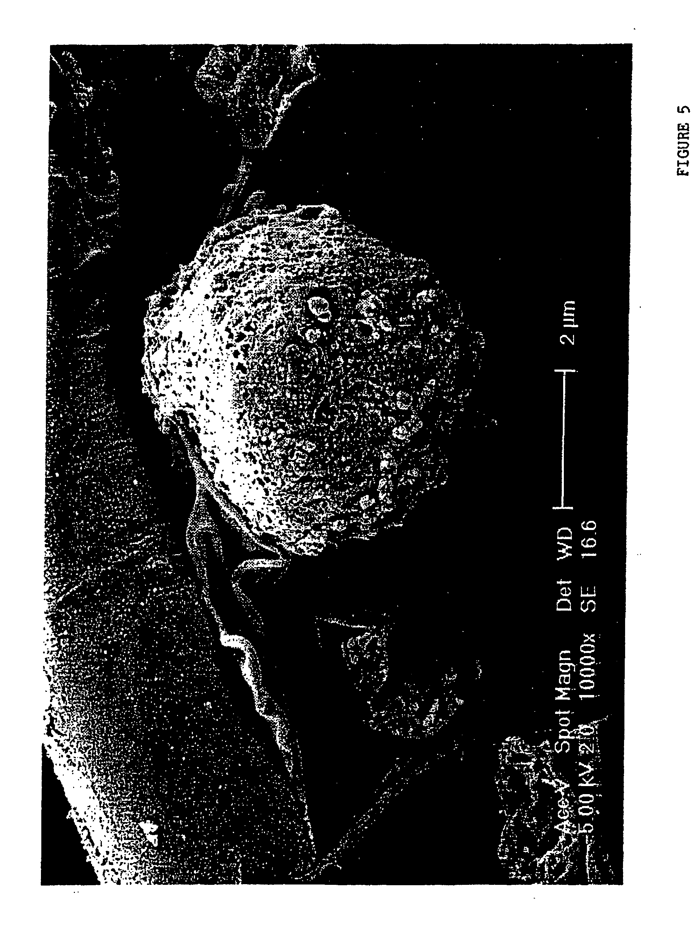

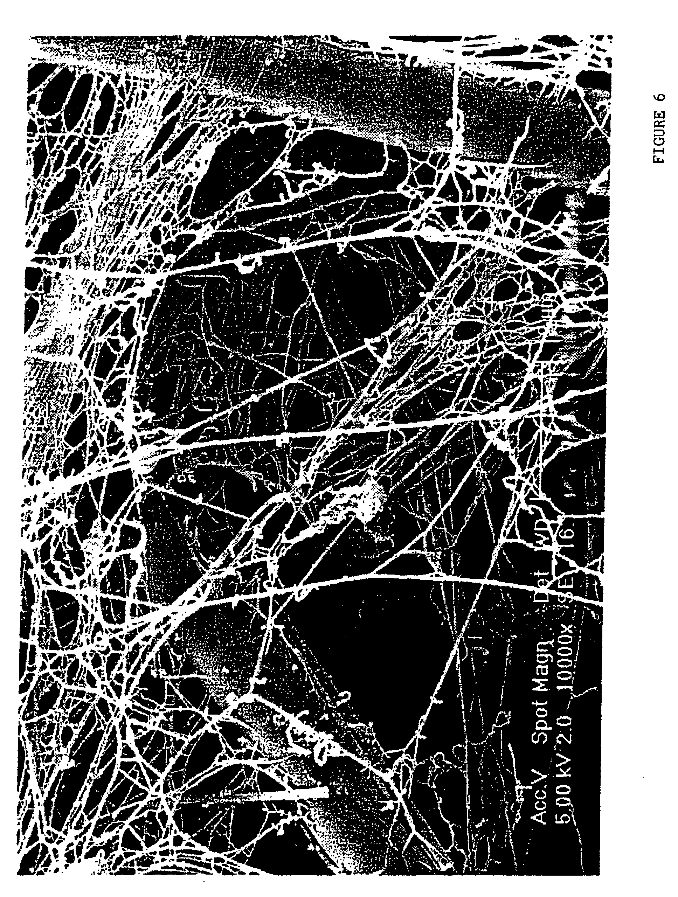

At various stages of the method, the filter and the retentate were analysed by scanning electron microscopy (SEM) in order to reveal the mechanism of cell retention, DNA retention and DNA release. Samples were fixed with 3% glutaraldehyde and 1% formaldehyde for 24 hours, washed with PIPES buffer, osmiated and gold treated with 25 nm of gold. The results are shown in Figures 1 to 9.

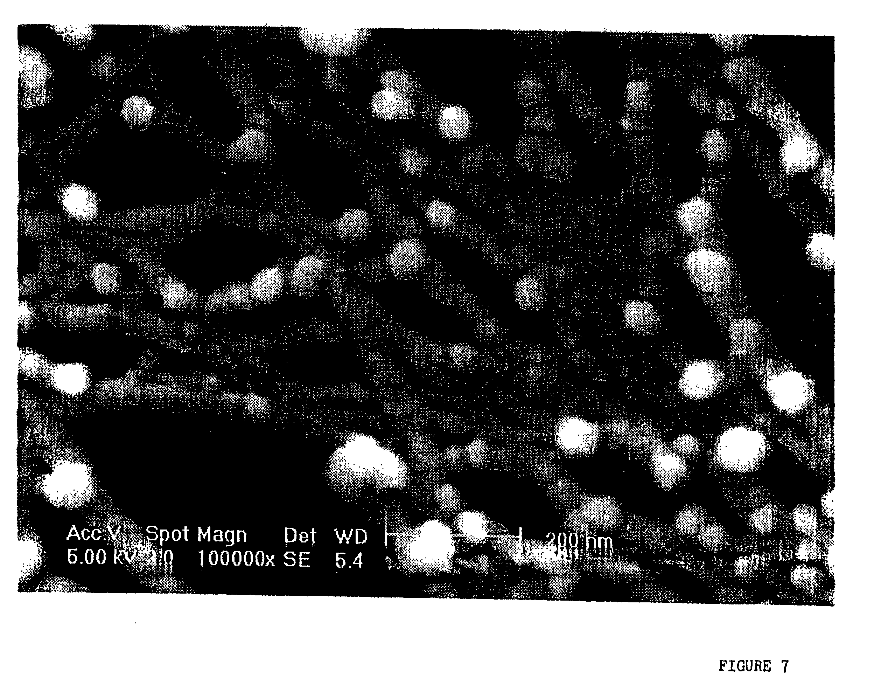

The strands shown by the image in Figure 7 are approximately 50 nm in diameter. It is thought that each strand is made up of a single DNA strand (less than 1 nm in thickness) and a 25 nm gold coating which encases each DNA strand.

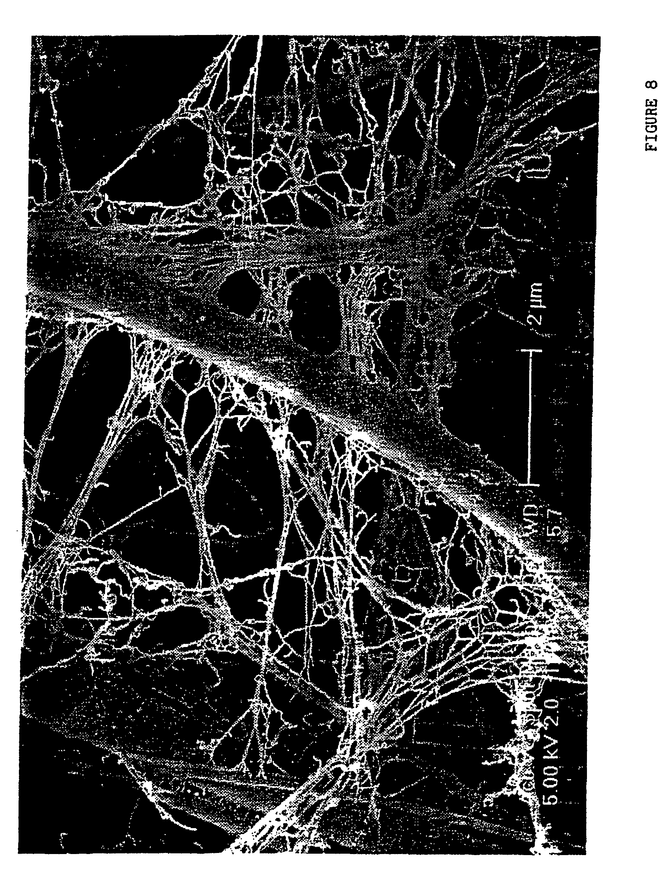

The image in Figure 8 shows that physically, the nucleic acid web appears to be unchanged after the heating and/or incubation step.

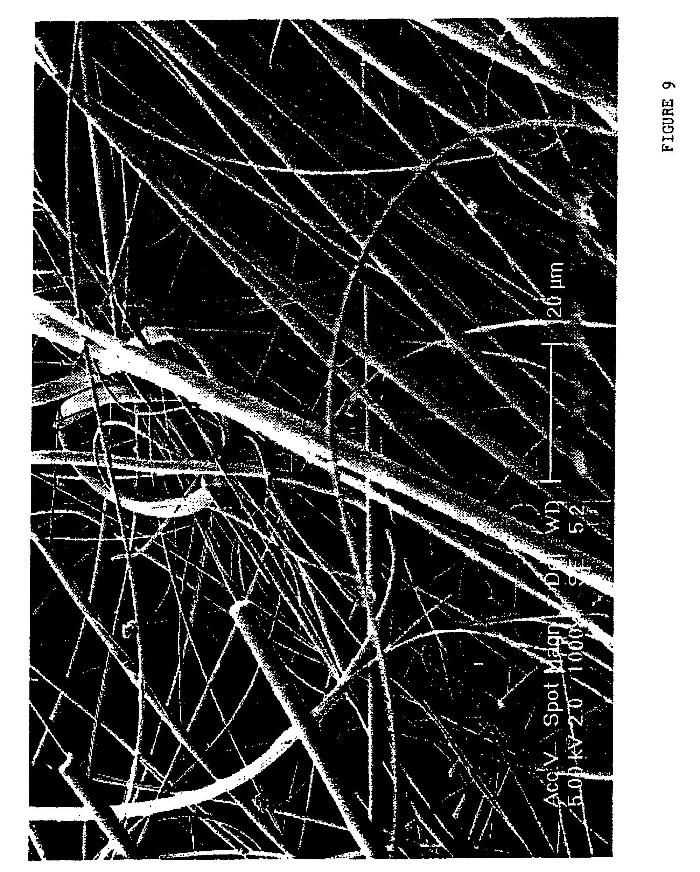

Figure 9 shows that the filter is very clean after elution of the nucleic acid.

Scanning spectroscopy analysis of the waste eluant in step 2 is a large absorbent peak at 410µm indicating the presence of haem. At the end of step 2, the retentate is on or in the filter.

Scanning spectroscopy analysis of the waste eluant from step 3 shows a large defined absorbance peak at 275µm indicating the presence of protein. A small peak at 410µm is visible indicating the presence of haem.

Scanning spectrophotometric analysis of the waste eluant from step 4 shows a very small protein peak. No haem peak is observed. No peak is observed at 260µm indicating that DNA is not present. This is confirmed also by agarose gel analysis.

Scanning spectrophotometric analysis of the final product shows a defined absorbance peak at 260µm indicating that DNA is present. The 260:280 ratio is approximately 1.8. When the DNA is eluted in water, absorbance between 200 and 230µm is zero indicating that there is no salt present.

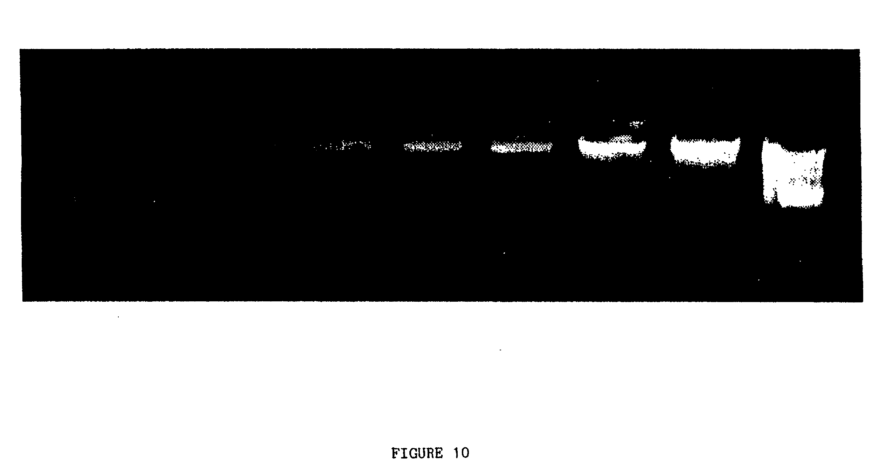

Restriction digestion tests suggest that the DNA recovered from this method is predominantly single stranded (see Figure 10).

### Example 2: Clinical Nucleic Acid Extraction Device

A device is provided which may, in one aspect, be used in the extraction of samples such as blood according to the method described above.

The device consists of the cartridge, which may be of any desired volume, typically 1ml, 5ml, or 10ml. The cartridge comprises a body which may be coated on its interior surface with a metal chelating agent such as EDTA. The cartridge has an inlet and an outlet disposed between which is a filter which may comprise a plurality of filters. The filter or filters are preferably disposed between a filter support or frit and a filter retaining member for retaining the filter or filters in place. The filter retaining member is preferably a ring which may make a friction fit inside the body of the cartridge. The body of the cartridge is preferably a barrel. The metal chelator acts to prevent coagulation of blood taken as a sample once inside the cartridge and other known anticoagulants may be used in its place. The inlet is preferably adapted to receive a needle assembly and may comprise a male lure. The outlet, typically arranged adjacent the filter support is preferably adapted to receive a syringe and is typically a female lure.

In a preferred arrangement, the filter or filters are as described above and may be used in the isolation of nucleic acid such as DNA as hereinbefore described.

In use, with a syringe and needle attached to the cartridge, the needle is inserted into a vein of a subject and blood drawn out by drawing back the syringe. Blood enters the cartridge preferably until it is full. The needle and syringe are then detached and the cartridge capped off with a suitable capping member. The cartridge may then be transported or stored at 4°C, -20°C or -70°C until required for processing. Storage conditions will vary depending on the likely length of time until DNA extraction may be performed.

When the DNA is to be extracted, frozen samples will need to be defrosted. The cartridge may be placed in a rack which may hold any number of samples, typically 96. The rack is then placed inside a device which, in sequence, delivers reagents and is heated to perform an extraction in accordance with the method described hereinbefore.

An advantage of this device is that the blood is collected, transported and extracted in a single device. This avoids the needs to transfer samples from collection to extraction device and minimises the potential for sample mix-up.

In a preferred embodiment, the device will bear a unique marking to identify the sample, such as a bar coding.

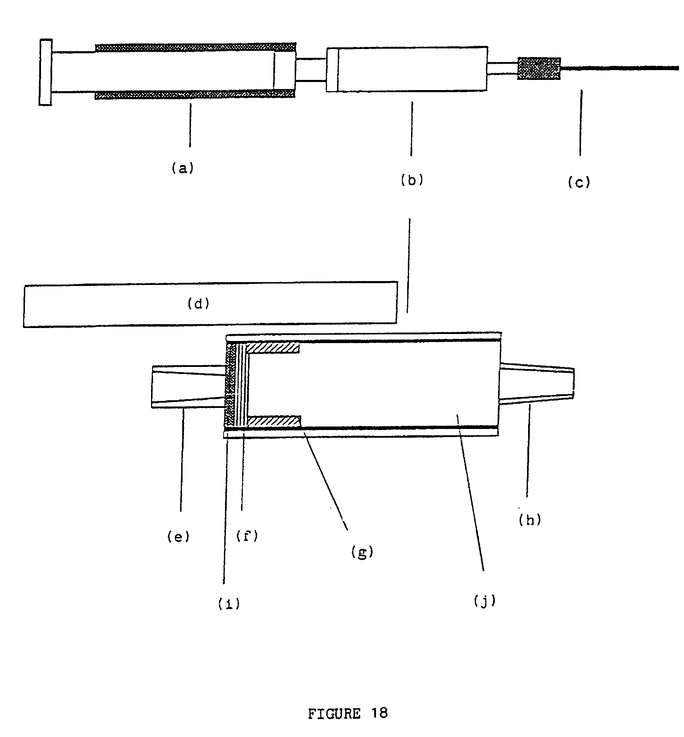

A specific example of the extraction device described above is shown in Figure 18.

### EXPERIMENTS

### Experiment 1: Filter Depth

It has hitherto been unknown to use a filter in the dual role of white cell capture and DNA capture. Once the white cells are lysed it is believed that the filter acts as a depth filter to the DNA. In order to explore this, the following investigation was undertaken.

A number of filters were stacked in an extraction column and DNA was isolated from 500µl of whole blood in accordance with the following protocol:
1) Add 500µl of red blood cell lysis solution to the blood and filter to waste.
2) Add 500µl 0.5% SDS solution and filter to waste.
3) Add 500µl 1mM Tris-HCl pH 8.5 and filter to waste.
4) Add 500µl 1mM Tris-HCl pH 8.5 and incubate for 50min at 82°C.
5) Filter and capture DNA eluant.

Prior to the final incubation and elution step the filters were removed and each one eluted and incubated individually. Fig 10 shows that there seems to be a gradient of DNA capture from the top filter to the bottom one. Lanes 1-8 show the recovery from filters from lowermost to uppermost respectively. This tends to indicate that the filter is physically retarding the DNA rather than binding it.

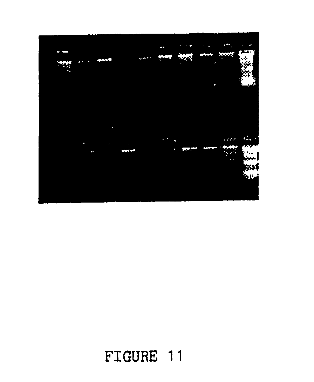

The experiment was repeated but this time small gaps were left at the edges of some of the filters. If the association between the DNA and the filter is entirely chemical then this would have no effect on the gradient of DNA quantity from the top to the bottom filter. Fig 11 shows that the filter fit should be carefully monitored in the method of the present invention since filters that are not true to the edge of the extraction vessel appear to bind much less DNA. The dropout in DNA levels on these filters had no effect on the capture of DNA filter below. This is further evidence that the method of the present invention involves the physical retardation of DNA rather than a chemical interaction.

### Experiment 2: Filter Depth

Analysis has shown that DNA recovery can be improved within the system by increasing the number of filters within the column. An experiment was performed according to the protocol below to assess the percentage of DNA captured by each subsequent layer of filter by processing whole blood using a 4-layered extraction column.

### Protocol:

1) Add 200µl of whole human blood to the 2ml-extraction vessel.
2) Add 1ml of RBCL and filter directly to waste.
3) Add a further 1ml of RBCL and filter directly to waste.
4) Add 1ml of 0.5% SDS and filter directly to waste.
5) Add 1ml of TE⁻¹ and filter directly to waste.
6) Add 100µl of TE⁻¹ and incubate at 90°C for 8 minutes.
7) Add 200µl of TE⁻¹ and collect.

The filters were removed prior to the elution step and the DNA collected off each filter separately. The most DNA was recovered from the uppermost filter and the least DNA was recovered from the lowest filter.

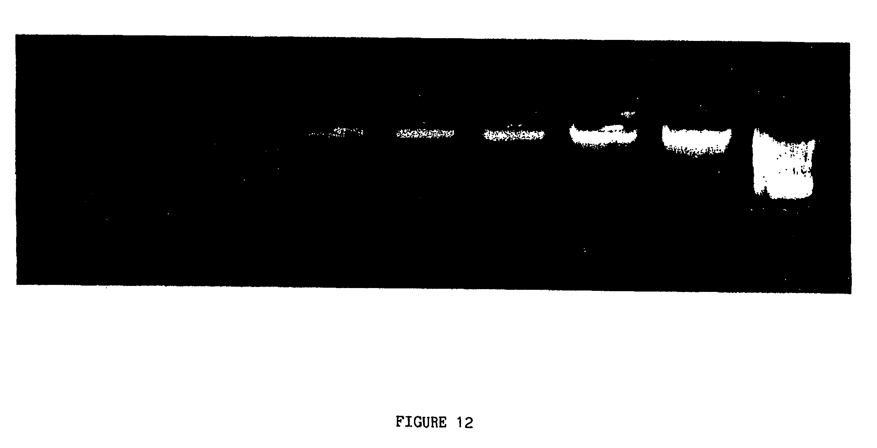

Figure 12 shows an agarose gel photograph showing the results of an experiment to show the proportion of DNA eluted from layers of filters within each system.

### Lane

- 1: 4th filter (Uppermost)
- 2: 3rd Filter
- 3: 2nd Filter
- 4: 1st Filter (Lowest)
- 5: Control
- 11: 1kb Ladder

### Experiment 3: Salt environment

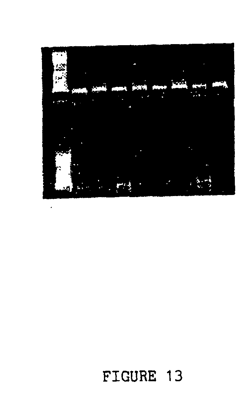

In the method according to the present invention, DNA remains bound to the silica during the wash steps in the presence of a 50% ethanol solution. In the method of the present invention DNA remains associated with the filter even in the presence of a low salt buffer. The upper agarose gel in Fig. 13 shows eight 500µl blood samples recovered by the method of the present invention using 1mM Tris pH 8.5 in the wash step. The lower gel shows recoveries using 50% Ethanol in the wash step. 20ul of DNA eluant was loaded in each lane.

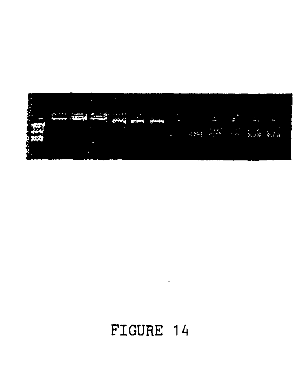

Further experiments using the method of the present invention have shown that DNA can be eluted off the filters in a high salt environment. Fig 14 shows DNA extracted from 100µl of whole blood and eluted in a range of salt concentrations. Lanes 1-3 were eluted in 1M KAc, lanes 4-6 in 0.1M, lanes 7-9 in 0.1M, and lanes 10-12 in 1mM. Each lane was loaded with 30µl of eluant.

### Experiment 4: Incubation Temperature

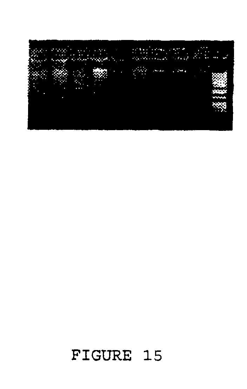

Temperature and time of incubation prior to elution can be advantageously controlled according to the present invention to enable a high yield of DNA to be obtained. Fig 15 shows the effect of temperature on DNA yield. Higher temperatures give higher yields and increased DNA shearing. Filters were incubated for 50 mins in water at 90°C (lanes 1-3), 85°C (4-6) and 80°C (7-9). 20ul of DNA eluant was loaded in each case.

### Experiment 5: Incubation Temperature and Incubation time

A number of experiments were carried out to establish the relationship between incubation time, incubation temperature and DNA yield and size. A standard 500µl extraction was executed according to the protocol of Experiment 2 except that incubation in step 6 was carried out over a range of times and temperatures.

**Table 3**

| Incubation temperature /°C | Time (hrs) | Yield (µg) | Mean size (kb) |
|---|---|---|---|
| 40 | 0.5 | 0 | - |
| 40 | 1.0 | 0 | - |
| 40 | 4.0 | 0.14 | >20kb |
| 40 | 24.0 | 2.0 | >20kb |
| 60 | 0.5 | 0.4 | >20kb |
| 60 | 1.0 | 0.1 | >20kb |
| 60 | 4.0 | 2.2 | >20kb |
| 60 | 24.0 | 12.0 | >20kb |
| 80 | 0.5 | 14.5 | >20kb |
| 80 | 1.0 | 25.8 | 10kb |
| 80 | 4.0 | 35.5 | 4kb |
| 80 | 24.0 | 40.4 | 0.5kb |
| 100 | 10 | 7.9 | >20kb |
| 100 | 20 | 14.0 | 5kb |
| 100 | 30 | 12.5 | 5kb |
| 100 | 40 | 16.5 | 3kb |
| 100 | 50 | - | - |
| 105 | 10 | 14.0 | >20kb |
| 105 | 20 | 14.0 | 5kb |
| 105 | 30 | 6.5 | 2kb |
| 105 | 40 | 6.0 | 1kb |
| 105 | 50 | 5.0 | 1kb |

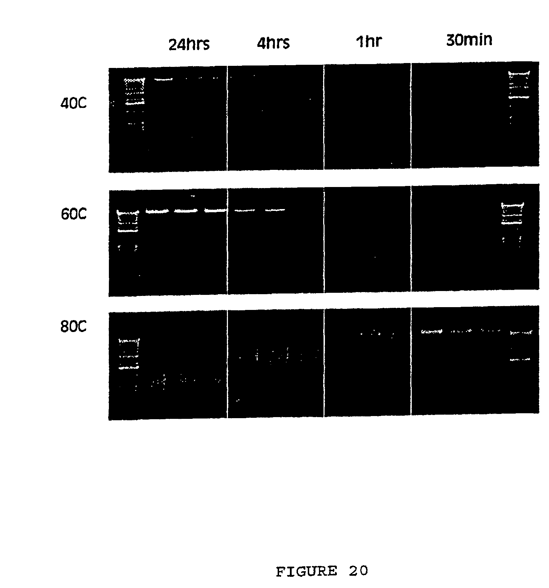

A small amount of DNA was obtainable from filters incubated at 40°C for 24 hours. Agarose gel analysis showed the DNA to be very large. At 60°C a small amount of very large DNA was obtained after 4 hours. At 80°C DNA was obtained after 30 minutes. More DNA was yielded over a longer time period however this was progressively more sheared (Figure 20).

At 105°C a high yield of DNA comes off the filter in 10 minutes. Any longer than this and the filter becomes visibly dry and the small amount of DNA that is recovered is severely sheared. Incubation at 100°C gives poor yields over short time periods and sheared DNA when incubated for longer.

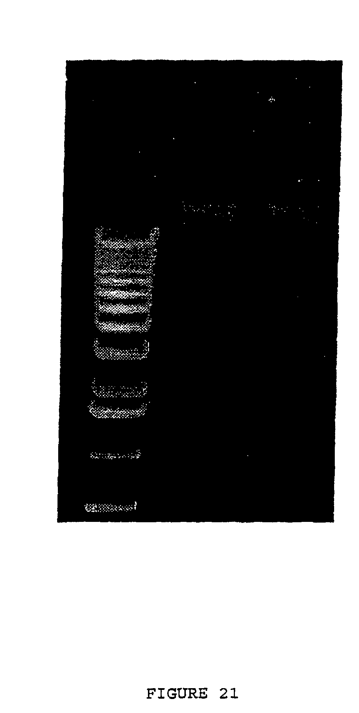

Work with clinical blood samples has shown that incubation at 90°C for 10 minutes gives a good balance between yield and DNA size giving 30-40µg of DNA per ml of whole blood. DNA has been shown to be approximately 85% > 40kb in size (Figure 21). Lane 1 in Figure 21 shows a 1kb extended ladder (largest band 40kb). Lanes 2 and 3 show 10µl of extracted DNA sample.

### Experiment 6: Heating step prior to elution

An experiment was carried out according to the protocol of Experiment 2 and 5:

Heat was applied to the system to initiate release of the DNA from the filter. The system allowed flexibility with respect to the duration of the heating step, as eluting 18 hours after the heating step resulted in the production of functional genomic DNA with only a small reduction in overall yield being recorded.

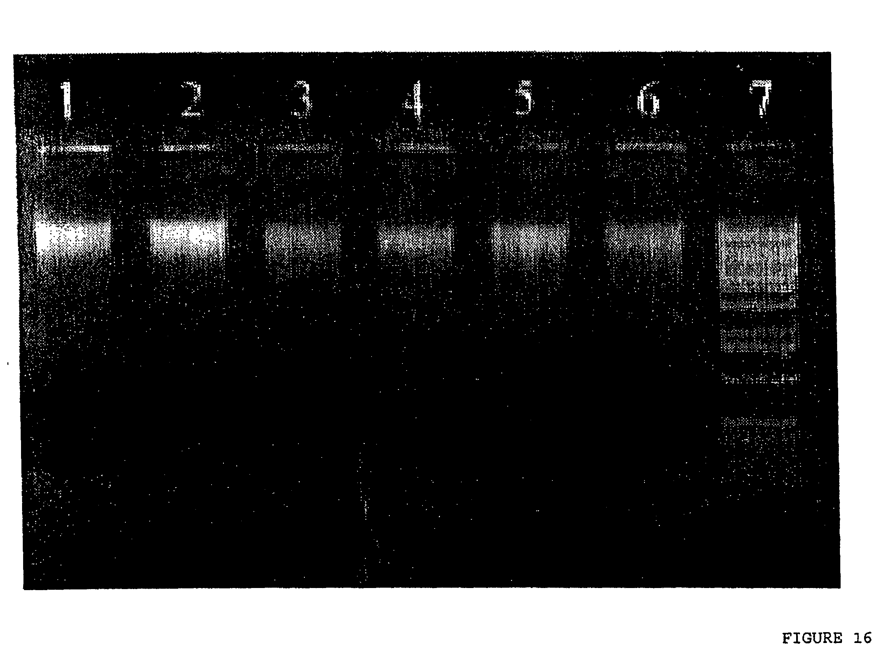

Figure 16 shows the comparisons between the time of elution post heating.

### Lane

- 2: heated to 90 °C, cooled for 8 hours and eluted at 37°C.
- 4: heated to 90°C, cooled for 1 hour and eluted.
- 6: heated to 90°C, eluted at 90°C.
- 7: 1kb ladder

### Experiment 7: Elution pH

An experiment was set up in accordance with the protocol of Experiments 2, 5 and 6. Ranges of elution buffers with different pH's were used to recover the DNA from the systems. The findings showed that DNA could be eluted over a wide pH range (pH5-pH11), covering both low and high salt buffers, suggesting no direct binding to the matrix.

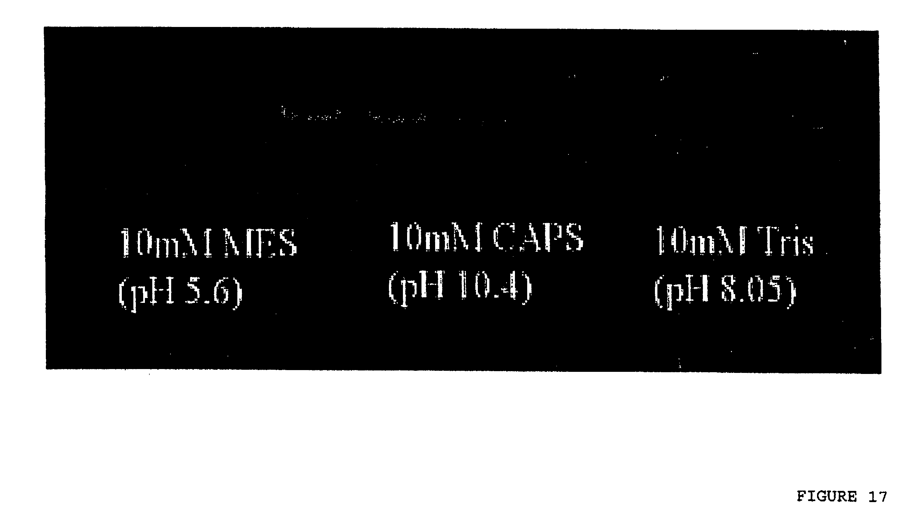

Figure 17 showing the results of altering the pH of the elution buffer over a range from pH5.5 to pH 11.0.

### Lane

- 1: 1kb Ladder
- 2: elution at pH 5.5
- 4: elution at pH 7.5
- 6: elution at pH 11.0

### Experiment 8: Elution Volume

An Experiment was conducted using the protocol of Experiments 2 and 5 to 8 except that a water eluant was used in step 7 at a volume varying from 100µl. The experiment was conducted using one and two filters. The results are shown in Table 5.

**Table 5**

| Number of Filters | Water added (µl) | Mean vol of recovered DNA soln (µl) | Mean DNA conc. (ng/µl) | Mean DNA Yield (ug) |
|---|---|---|---|---|
| 1 | 100 | 168 | 67 | 11.3 |
| 1 | 200 | 254 | 50 | 12.8 |
| 1 | 300 | 358 | 36 | 12.9 |
| 1 | 400 | 455 | 28 | 12.9 |
| 2 | 100 | 170 | 56 | 9.5 |
| 2 | 200 | 252 | 49 | 12.5 |
| 2 | 300 | 356 | 36 | 13.15 |
| 2 | 400 | 469 | 33 | 15.5 |

Optimum yields and concentrations are obtained with the addition of 200µl of eluant in a single filter column. Very slightly higher DNA yields can be obtained in a two-filter system or with higher volumes of eluant at the expense of DNA concentrations.

### Experiment 9: PCR Analysis of Purified Product

Analysis of the final solution included repeating the Qiagen PCR assay. This assay amplifies a lkb-region using increasing volumes of DNA in a set 50µl reaction. Although this does not result in equal masses of DNA being added, the aim of this experiment is to determine if any background inhibitors are present. Reactions were set up as shown in Table 4.

**Table 4**

| Reaction | DNA Control | Present DNA Product | Buffer (15 mM Mg) 10 times | dNTPs | Prima 1 | Prima 2 | Taq Polymerase | Water |
|---|---|---|---|---|---|---|---|---|
| 1 | 0 | 0 | 5 | 5 | 0.2 | 0.2 | 0.5 | 39.1 |
| 2 | 0 | 1 | 5 | 5 | 0.2 | 0.2 | 0.5 | 38.1 |
| 3 | 0 | 5 | 5 | 5 | 0.2 | 0.2 | 0.5 | 34.1 |
| 4 | 0 | 10 | 5 | 5 | 0.2 | 0.2 | 0.5 | 29.1 |
| 5 | 0 | 15 | 5 | 5 | 0.2 | 0.2 | 0.5 | 24.1 |
| 6 | 0 | 20 | 5 | 5 | 0.2 | 0.2 | 0.5 | 19.1 |
| 7 | 5 | 0 | 5 | 5 | 0.2 | 0.2 | 0.5 | 34.1 |

The samples were amplified using the standard service amplification procedure. All the reactions appeared to work in this assay. Therefore, it was concluded that the present DNA product is capable of amplification.

### Experiment 10: SDS Treated Filter (Comparative Experiment)

100ml of 10% SDS was dried onto Whatman GF/D filters on a hot block. 200µl whole blood was added to the column, incubated for 1 minute and then eluted to waste. Then, the column was rinsed with 2ml of water and, again, eluted to waste. In all experiments, there was haem still visible on the filter at the end of the experiment. Redness was visible in all final eluants. All final eluants were frothy indicating the presence of SDS. From these experiments, it was apparent that forcing DNA through SDS treated filters seems to cause extensive DNA shearing. The number of filters (i.e. the depth of the filter) seemed to have no noticeable affect on this.

### Experiment 11: Whatman GF/C Filter (Comparative Experiment)

It was found that the composition and dimensions of the Whatman GF/C filters were not suitable for the retention of cells and nucleic acid. The General Protocol set out above was replaced, but this time the stack of 4 GF/D variant filters was replaced with a stack of 4 GF/C filters. 500µl whole blood was added to the column, followed by 500µl red blood cell lysis solution. An attempt was made to filter the filtrate to waste, however, the filter became blocked almost immediately. It was apparent that the dimensions of the GF/C filter do not enable the retention cells therein. It is believed that the GF/C filter acts as an absolute barrier to the cells in the absence of a chaotrope.]

## Claims

1. A method for isolating DNA from a nucleated cell, which comprises:
(a) applying a sample comprising cells containing DNA to a filter, whereby the cells are retained as a retentate and contaminants are removed;
(b) lysing the retentate from step (a) whilst the retentate is retained by the filter to form a cell lysate containing the DNA;
(c) filtering the cell lysate with the filter to retain the DNA and remove remaining cell lysate;
(d) optionally washing the DNA retained by the filter; and
(e) eluting the DNA,
wherein the filter composition and dimensions are selected so that (i) the filter is capable of retaining the cells and the DNA and (ii) the DNA is retained by the filter in step (c) substantially in the absence of ionic interaction.

2. A method according to claim 1, wherein step (b) comprises lysing the retentate whilst it is entrapped within the filter.

3. A method according to claim 1 or claim 2, wherein the retentate comprises condensed nuclear material and cell debris.

4. A method according to any one of claims 1 to 3, wherein the retentate comprises intact whole cells.

5. A method according to any one of the preceding claims, wherein step (b) comprises lysing the retentate to form a cell lysate containing the DNA by the addition of a low salt buffer.

6. A method according to claim 4 or claim 5, wherein step (b) comprises:
(b1) rupturing the intact whole cells retained by the filter to leave condensed nuclear material which also is retained by the filter; and
(b2) lysing the condensed nuclear material to form a cell lysate containing the DNA.

7. A method according to claim 6, wherein the intact whole cells are ruptured to form condensed nuclear material by the addition of detergent.

8. A method according to claim 7, wherein the detergent is SDS, TWEEN 20 or LDS.

9. A method according to any one of the preceding claims, wherein the filter composition and dimensions are selected so that the DNA is retained by the filter in step (c) by a physical retarding of the movement of the DNA through the filter.

10. A method according to any one of the preceding claims, wherein the filter composition and dimensions are selected so that the DNA is retained by the filter in step (c) in the form of a web.

11. A method according to any one of the preceding claims wherein the filter comprises a plurality of fibres and has a substantially disordered structure.

12. A method according to claim 11, wherein the fibre diameters are selected so that the DNA is retained by the filter in step (c) in the form of a web.

13. A method according to claim 12, wherein the fibre diameters are in the range of from 1µm to 10µm.

14. A method according to any one of the preceding claims wherein the filter composition and dimensions are selected so that the filter is substantially incapable of retaining purified DNA when purified DNA is applied thereto.

15. A method according to any one of the preceding claims, wherein the filter composition and dimensions are selected so that the filter is capable of retaining the cells and the DNA substantially in the absence of a chaotrope.

16. A method according to any one of the preceding claims, wherein the filter comprises a silica-based filter or a plastics-based filter.

17. A method according to any one of the preceding claims, wherein the filter comprises a plurality of filters arranged in series.

18. A method according to claim 17, wherein the plurality of filters is stacked one above the other and supported on a frit.

19. A method according to any one of the preceding claims, wherein the DNA is heated to an elevated temperature, whilst retained by the filter prior to eluting in step (e).

20. A method according to claim 19, wherein the elevated temperature is in the range 40°C to 125°C.

21. A method according to claim 20, wherein the elevated temperature is in the range 80°C to 95°C.

22. A method according to any one of the preceding claims, wherein the cells comprise white blood cells, epithelial cells, buccal cells, tissue culture cells or colorectal cells.

23. A method according to claim 22, wherein the cells are white blood cells, which method further comprises applying whole blood to the solid phase, optionally lysing the red blood cells therefrom, optionally washing the solid phase to remove contaminants and obtaining the cell lysate from the white blood cells.

24. A method according to any one of the preceding claims which is carried out without any centrifugation steps.

25. A method according to any one of the preceding claims, which is carried out substantially in the absence of a chaotrope.

## Patentansprüche

1. Verfahren zum Isolieren von DNA aus einer kernhaltigen Zelle, umfassend
(a) das Auftragen einer Probe, welche DNA enthaltende Zellen umfaßt, auf einen Filter, wobei die Zellen als ein Rückstand zurückbehalten werden und Verunreinigungen entfernt werden,
(b) das Lysieren des Rückstandes aus Schritt (a), während der Rückstand durch den Filter zurückgehalten wird, um ein DNA enthaltendes Zell-Lysat zu bilden,
(c) das Filtrieren des Zell-Lysates mit dem Filter, um die DNA zurückzuhalten und verbleibendes Zell-Lysat zu entfernen,
(d) gegebenenfalls das Waschen der durch den Filter zurückgehaltenen DNA, und
(e) das Eluieren der DNA,
wobei die Filterzusammensetzung und Abmessungen derart ausgewählt sind, daß (i) der Filter befähigt ist, die Zellen und die DNA zurückzuhalten, und (ii) die DNA durch den Filter in Schritt (c) im wesentlichen in Abwesenheit einer ionischen Wechselwirkung zurückgehalten wird.

2. Verfahren nach Anspruch 1, wobei der Schritt (b) das Lysieren des Rückstandes umfaßt, während dieser innerhalb des Filters eingeschlossen ist.

3. Verfahren nach Anspruch 1 oder 2, wobei der Rückstand kondensiertes Kernmaterial und Zellbruchstücke umfaßt.

4. Verfahren nach einem der Ansprüche 1 bis 3, wobei der Rückstand intakte ganze Zellen umfaßt.

5. Verfahren nach einem der vorhergehenden Ansprüche, wobei der Schritt (b) das Lysieren des Rückstandes durch Zugabe eines Niedrigsalzpuffers umfaßt, um ein DNA enthaltendes Zell-Lysat zu bilden.

6. Verfahren nach Anspruch 4 oder 5, wobei der Schritt (b)
(b1) das Aufbrechen der durch den Filter zurückgehaltenen intakten ganzen Zellen, um kondensiertes Kernmaterial, welches auch durch den Filter zurückgehalten wird, zurückzulassen, und
(b2) das Lysieren des kondensierten Kemmaterials, um ein die DNA enthaltendes Zell-Lysat zu bilden, umfaßt.

7. Verfahren nach Anspruch 6, wobei die intakten ganzen Zellen durch die Zugabe eines Detergents aufgebrochen werden, um kondensiertes Kernmaterial zu bilden.

8. Verfahren nach Anspruch 7, wobei das Detergents SDS, TWEEN 20 oder LDS ist.

9. Verfahren nach einem der vorherigen Ansprüche, wobei die Filterzusammensetzung und Abmessungen derart ausgewählt sind, daß die DNA durch den Filter (c) mittels physikalischen Retardierens der Bewegung der DNA durch den Filter zurückgehalten wird.

10. Verfahren nach einem der vorherigen Ansprüche, wobei die Filterzusammensetzung und die Abmessungen derart ausgewählt sind, daß die DNA durch den Filter in Schritt (c) in Form eines Netzes zurückgehalten wird.

11. Verfahren nach einem der vorherigen Ansprüche, wobei der Filter eine Vielzahl von Fasern umfaßt und eine im wesentlichen ungeordnete Struktur aufweist.

12. Verfahren nach Anspruch 11, wobei die Faserdurchmesser derart ausgewählt sind, daß die DNA durch den Filter in Schritt (c) in Form eines Netzes zurückgehalten wird.

13. Verfahren nach Anspruch 12, wobei die Faserdurchmesser im Bereich von 1 µm bis 10 µm liegen.

14. Verfahren nach einem der vorherigen Ansprüche, wobei die Filterzusammensetzung und Abmessungen derart ausgewählt sind, daß der Filter im wesentlichen unfähig ist, gereinigte DNA zurückzuhalten, wenn gereinigte DNA darauf aufgetragen wird.

15. Verfahren nach einem der vorherigen Ansprüche, wobei die Filterzusammensetzung und Abmessungen derart ausgewählt sind, daß der Filter befähigt ist, die Zellen und die DNA im wesentlichen in Abwesenheit eines chaotropen Mittels zurückzuhalten.

16. Verfahren nach einem der vorherigen Ansprüche, wobei der Filter einen Filter auf Siliziumdioxidbasis oder einen Filter auf Kunststoffbasis umfaßt.

17. Verfahren nach einem der vorherigen Ansprüche, wobei der Filter eine Vielzahl von in Reihe angeordneten Filtern umfaßt.

18. Verfahren nach Anspruch 17, wobei die Vielzahl der Filter übereinander gestapelt ist und durch eine Fritte getragen wird.

19. Verfahren nach einem der vorherigen Ansprüche, wobei die DNA auf eine erhöhte Temperatur erwämt wird, während sie durch den Filter vor der Elution in Schritt (e) zurückgehalten wird.

20. Verfahren nach Anspruch 19, wobei die erhöhte Temperatur im Bereich von 40°C bis 125°C liegt.

21. Verfahren nach Anspruch 20, wobei die erhöhte Temperatur im Bereich 80°C bis 95°C liegt.

22. Verfahren nach einem der vorherigen Ansprüche, wobei die Zellen weiße Blutkörperchen, Epithelzellen, Bukkalzellen, Gewebekulturzellen oder Colorektalzellen umfassen.

23. Verfahren nach Anspruch 22, wobei die Zellen weiße Blutkörperchen sind, und wobei das Verfahren ferner das Auftragen von Vollblut auf die feste Phase, gegebenenfalls das Lysieren der roten Blutkörperchen davon, gegebenenfalls das Waschen der festen Phase, um Verunreinigungen zu entfernen, und das Erhalten des Zell-Lysats aus den weißen Blutkörperchen umfaßt.

24. Verfahren nach einem der vorherigen Ansprüche, welches ohne irgendwelche Zentrifugationsschritte durchgeführt wird.

25. Verfahren nach einem der vorherigen Ansprüche, welches im wesentlichen in Abwesenheit eines chaotropen Mittels durchgeführt wird.

## Revendications

1. Procédé d'isolement d'ADN à partir d'une cellule nucléée, comprenant:
(a) l'application d'un échantillon refermant des cellules contenant l'ADN sur filtre, de sorte que les cellules sont retenues sous forme de rétentat et que les contaminants sont éliminés;
(b) la lyse du rétentat de l'étape (a) alors que le rétentat est retenu par le filtre, pour former un lysat cellulaire contenant l'ADN;
(c) la filtration du lysat cellulaire à l'aide du filtre pour retenir l'ADN et éliminer le lysat cellulaire restant;
(d) éventuellement, le lavage de l'ADN retenu sur le filtre; et
(e) l'élution de l'ADN,
la composition et les dimensions du filtre étant choisies de manière à ce que (i) le filtre puisse retenir les cellules et l'ADN et que (ii) l'ADN soit retenu par le filtre dans l'étape (c) essentiellement en l'absence d'interaction ionique.

2. Procédé selon la revendication 1, dans lequel l'étape (b) comprend la lyse du rétentat alors que celui-ci est piégé dans le filtre.

3. Procédé selon la revendication 1 ou la revendication 2, dans lequel le rétentat comprend du matériel nucléaire condensé et des débris cellulaires.

4. Procédé selon l'une quelconque des revendications 1 à 3, dans lequel le rétentat comprend des cellules entières intactes.

5. Procédé selon l'une quelconque des revendications précédentes, dans lequel l'étape (b) comprend la lyse du rétentat pour former un lysat cellulaire contenant l'ADN par addition d'un tampon à faible teneur en sel.

6. Procédé selon la revendication 4 ou la revendication 5, dans lequel l'étape (b) comprend:
(b1) la rupture des cellules entières intactes retenues par le filtre pour laisser le matériel nucléaire condensé qui est aussi retenu par le filtre; et
(b2) la lyse du matériel nucléaire condensé pour former un lysat cellulaire contenant l'ADN.

7. Procédé selon la revendication 6, dans lequel les cellules entières intactes sont rompues pour obtenir du matériel nucléaire condensé par addition de détergent.

8. Procédé selon la revendication 7, dans lequel le détergent est le SDS, le TWEEN 20 ou le LDS.

9. Procédé selon l'une quelconque des revendications précédentes, dans lequel la composition et les dimensions du filtre sont choisies de manière à ce que l'ADN soit retenu par le filtre dans l'étape (c) par un retardement physique du mouvement de l'ADN à travers le filtre.

10. Procédé selon l'une quelconque des revendications précédentes, dans lequel la composition et les dimensions du filtre sont choisies de manière à ce que l'ADN soit retenu par le filtre dans l'étape (c) sous la forme d'une nappe.

11. Procédé selon l'une quelconque des revendications précédentes, dans lequel le filtre comprend un pluralité de fibres et possède une structure essentiellement désordonnée.

12. Procédé selon la revendication 11, dans lequel les diamètres des fibres sont choisis de manière à ce que l'ADN soit retenu par le filtre dans l'étape (c) sous la forme d'une nappe.

13. Procédé selon la revendication 12, dans lequel les diamètres des fibres sont compris dans l'intervalle de 1 µm à 10 µm.

14. Procédé selon l'une quelconque des revendications précédentes, dans lequel la composition et les dimensions du filtre sont choisies de manière à ce que le filtre ne puisse pas sensiblement retenir l'ADN purifié lorsque de l'ADN purifié y est appliqué.

15. Procédé selon l'une quelconque des revendications précédentes, dans lequel la composition et les dimensions du filtre sont choisies de manière à ce que le filtre puisse retenir les cellules et l'ADN essentiellement en l'absence d'un chaotrope.

16. Procédé selon l'une quelconque des revendications précédentes, dans lequel le filtre comprend un filtre à base de silice ou un filtre à base de matières plastiques.

17. Procédé selon l'une quelconque des revendications précédentes, dans lequel le filtre comprend une pluralité de filtres disposés en série.

18. Procédé selon la revendication 17, dans lequel la pluralité de filtres est empilée l'un sur l'autre et supporté sur une fritte.

19. Procédé selon l'une quelconque des revendications précédentes, dans lequel l'ADN est chauffé à une température élevée, alors qu'il est retenu par le filtre avant l'élution de l'étape (e).

20. Procédé selon la revendication 19, dans lequel la température élevée est comprise dans l'intervalle de 40°C à 125°C.

21. Procédé selon la revendication 20, dans lequel la température élevée est comprise dans l'intervalle de 80°C à 95°C.

22. Procédé selon l'une quelconque des revendications précédentes, dans lequel les cellules comprennent des globules blancs, des cellules épithéliales, des cellules buccales, des cellules de culture tissulaire ou des cellules colo-rectales.

23. Procédé selon la revendication 22, dans lequel les cellules sont des globules blancs, lequel procédé comprend en outre l'application du sang entier à la phase solide, éventuellement la lyse des globules rouges à partir du sang entier, éventuellement le lavage de la phase solide pour éliminer les contaminants et l'obtention du lysat cellulaire à partir des globules blancs.

24. Procédé selon l'une quelconque des revendications précédentes, qui est réalisé sans aucune étape de centrifugation.

25. Procédé selon l'une quelconques des revendications précédentes, qui est réalisé essentiellement en l'absence d'un chaotrope.
